# DEMANDE DE BREVET EUROPEEN

(11) **EP 1 566 450 A2**
(43) Date de publication de la demande: **24.08.2005**
(21) Numéro de dépôt: 04011368.0
(22) Date de dépôt: 23.11.1998
(51) Int. Cl.: C12Q 1/68

(54) **Réactif et methode pour la détection du gene 17.5 lié au CMH d'oiseaux d'élevage**

(30) Priorité: 21.11.1997 FR 9714669
(62) Demande divisionnaire de: 98955723.6
(71) Demandeur: CENTRE NATIONAL DE LA RECHERCHE SCIENTIFIQUE (CNRS), 75794 Paris Cedex 16 (FR); INSTITUT NATIONAL DE LA RECHERCHE AGRONOMIQUE (INRA), 75341 Paris Cédéx 07 (FR)
(72) Inventeur: Zoorob, Rima, 93160 Noisy-le-Grand (FR); Auffray, Charles, 94370 Sucy-en-Brie (FR); Chaussee, Anne-Marie, 37380 Saint-Laurent-en-Gatines (FR)
(74) Mandataire: Peaucelle, Chantal

(57) **Abrégé**

L'invention a pour objet une méthode de génotypage d'oiseaux d'élevage, permettant de contrôler les poulets résistants aux maladies viro-induites, caractérisée en ce qu'elle comprend
- l'amplification d'un échantillon d'acide nucléique provenant de l'animal à étudier à l'aide d'un ou de plusieurs couples d'amorces capables de s'hybrider spécifiquement avec l'acide nucléique d'une région polymorphe du système Rfp-Y du CMH desdits oiseaux, ces amorces étant élaborées à partir des molécules d'acides nucléiques du gène *17.5*, et
la détection des produits de PCR obtenus.

## Description

L'invention a pour objet la détection de gènes liés au complexe majeur d'histocompatibilité (CMH) d'oiseaux d'élevage, tels que le poulet. A ce titre, elle concerne les applications de molécules d'acides nucléiques permettant de détecter ceux des gènes du CMH impliqués dans les phénomènes de résistance ou de susceptibilité au développement de tumeurs viro-induites. Elle concerne en particulier les applications de ces molécules d'acides nucléiques, notamment pour le développement de tests de génotypage chez les oiseaux d'élevage, en particulier le poulet, et pour la sélection d'animaux d'intérêt.

Les maladies virales infectieuses sont redoutées des éleveurs en raison de leur caractère contagieux qui conduit à des pertes importantes d'animaux.

La vaccination a constitué une prophylaxie efficace jusqu'à l'émergence de souches hypervirulentes, rendant nécessaire l'identification des haplotypes résistants.

Diverses méthodes ont ainsi été proposées pour tenter de sélectionner ceux des animaux qui sont capables de résister à de telles pathologies et ceux qui sont au contraire susceptibles d'être affectés.

Les techniques les plus utilisées en routine sont basées sur des polymorphismes sérologiques ou de type RFLP. Toutefois, ces méthodes ne fournissent pas de connaissances précises sur le phénomène de résistance ou de susceptibilité à la maladie, en particulier par manque de caractère discriminant vis-à-vis des gènes des systèmes B ou Rfp-Y du CMH.

Divers travaux ont porté sur le positionnement de gènes du CMH de poulet.

Dans l'article de GUILLEMOT et al, EMBO Journal, 1988, Vol.7, N°9, p 2775-2785, les auteurs dont certains sont co-inventeurs, rapportent la cartographie des gènes β de classe I, chez le poulet, et décrivent 5 gènes de classe II (B-L).

Dans Immunogenetics 1994, 39 :71-73, les auteurs rapportent des régions d'homologie entre Rfp-Y et les gènes du CMH-B.

Immunogenetics 1996, 44: 242-245, porte sur l'étude de l'association entre l'haplotype Rfp-Y et la maladie de Marek chez le poulet. Cet article indique qu'un second complexe d'histocompatibilité de gènes a été mis en évidence chez le poulet, à savoir le complexe Rfp-Y qui comprend des gènes de CMH de classe I et de classe II. Mais ces deux documents ne décrivent pas de gènes séquencés utilisables pour le génotypage de poulet.

De même dans Immunogenetics 1994, 39 : 221-229, les auteurs, dans certains sont co-inventeurs, rapportent le lien entre un nouveau membre de la famille du supergène de la lectine et les gènes CMH de poulet.

Eur. J. Immunol. 1993, 23: 1139-1145, dans lequel certain des co-inventeurs sont également co-auteurs, décrit 5 gènes B-LB du poulet appartenant à deux familles différentes.

The Journal of Immunology, 1992, Vol. 148, 1532-1546, se rapporte à des études comparatives préliminaires concernant les séquences BF du poulet.

Les travaux des inventeurs sur le séquençage de gènes du CMH a montré la complexité génétique de cette région, ce qui les a conduits à prendre en compte un autre type de polymorphisme, à savoir basé sur la séquence de ces gènes et des régions apparentées, telles que celles de leurs promoteurs et des régions microsatellitaires. Les inventeurs ont ainsi mis au point des moyens pour disposer de molécules oligonucléotidiques hautement spécifiques des polymorphismes observés, permettant d'identifier les parties de gènes, et même les sites impliqués dans le contrôle de la résistance ou de la susceptibilité au développement de tumeurs.

Le caractère spécifique de ces molécules, vis-à-vis d'un gène donné de l'un des systèmes du CMH, en fait des outils discriminants particulièrement fiables pour identifier avec précision la capacité de résistance ou de susceptibilité du poulet étudié, ou d'autres oiseaux, à une infection virale, et pour étudier au niveau moléculaire les séquences du CMH impliquées.

L'invention a donc pour but de fournir une méthode et un kit de détection de génotypes de mise en oeuvre aisée en routine.

Les molécules d'acides nucléiques utilisées dans la méthode de génotypage selon l'invention sont des molécules, isolées de leur environnement naturel, d'acides nucléiques du gène 17.5 codant pour une protéine impliquée dans le contrôle de la résistance ou de la susceptibilité au développement de tumeurs viro-induites chez les oiseaux d'élevage, telles que celles de la maladie de Marek chez le poulet, avec le cas échéant, les régions qui leur sont attachées, telles que celles du promoteur ou microsatellitaires. Le terme gène tel qu'utilisé dans la description et les revendications englobe ces régions.

Elle concerne également l'utilisation des molécules d'acides nucléiques capable de s'apparier avec l'un des brins du gène 17.5 dans des conditions faiblement stringentes.

L'appariement dans des conditions de faible stringence auquel il est fait référence ci-dessus est réalisé à température ambiante, dans un milieu 0,1 SSC, avec lavage à température ambiante.

Le gène 17.5 appartient à la superfamille des gènes codant pour les lectines. Ce gène est décrit dans Immunogenetics 39:221-229, 1994.

L'étude des séquences d'acides nucléiques des molécules définies plus haut a permis de repérer avec précision les blocs de polymorphismes qui doivent être détectés pour établir un génotypage fiable et précis.

En comparant les séquences de ces blocs, provenant de différents gènes d'un même haplotype ou d'un même gène de différents haplotypes, les inventeurs ont pris en considération les enchaînements divergents et élaboré, pour chaque gène, des oligonucléotides complémentaires de ces enchaînements divergents.

On dispose ainsi d'amorces spécifiques et discriminantes vis-à-vis du gène 17.5.

L'invention vise tout spécialement l'utilisation dans la méthode de génotypage de molécules d'oligonucléotides correspondant à ces enchaînements du gène 17.5 et comprenant une partie de la région polymorphe des systèmes du CMH du poulet ou autres oiseaux d'élevage.

On rappelle que la région polymorphe peut être dans le gène ou dans une région apparentée telle que les régions microsatellitaires ou celle du promoteur.

Selon un mode de réalisation de l'invention, les polymorphismes sont liés à la fonction des systèmes du CMH.

Il s'agit ainsi avantageusement de molécules correspondant à une partie d'un exon.

Selon un mode de réalisation de l'invention, les molécules oligonucléotidiques mises en oeuvre correspondent à une partie d'une région polymorphe qui n'est pas liée à la fonction des systèmes du CMH. Des régions préférées de ce type sont des microsatellites.

Avec les molécules oligonucléotidiques définies à partir du gène 17.5, mais selon la démarche de l'invention, on dispose de jeux d'amorces hautement spécifiques, permettant de déterminer avec précision l'haplotype de l'animal à étudier et de détecter s'il est résistant au développement de tumeurs viro-induites, ou au contraire susceptible d'être affecté.

L'invention vise donc une méthode de génotypage d'oiseaux d'élevage et notamment du poulet.

Cette méthode est caractérisée en ce qu'elle comprend
- l'amplification d'un échantillon d'acide nucléique provenant de l'animal à étudier à l'aide d'un ou de plusieurs couples d'amorces capables de s'hybrider spécifiquement avec le gène 17.5, et
- la détection des produits de PCR obtenus.

Une simple comparaison des résultats obtenus avec un référentiel établi au préalable permet de déterminer rapidement l'haplotype de l'animal.

L'échantillon d'acide nucléique est constitué en particulier par de l'ADN génomique extrait de matériel biologique de l'animal à étudier ou par ce matériel même, en particulier par du sang de l'animal. Il peut s'agir en variante d'ADNc, d'ARN ou encore de PNA (polypeptides nucleic acids).

Les amorces sont élaborées à partir des molécules oligonucléotidiques du gène 17.5 codant pour une protéine impliquée dans le contrôle de la résistance ou de la susceptibilité aux tumeurs viro-induites chez les oiseaux d'élevage et notamment de poulet.

Il s'agit par exemple d'amorces permettant de détecter des haplotypes du système RFp-Y, et élaborées à partir du gène 17.5, comme le couple :
17.52 : CAG GAT CTG CAC TGG CCA ATA
17.5, R1 : GAA TGG CGG TGC TTC CGT GCC TGG

La détection des produits de PCR est effectuée selon les techniques classiques. Ces techniques comprennent le séquençage, l'électrophorèse, les hybridations avec analyse SSOP ou SSCP.

Cette technique sera avantageusement choisie selon la nature du polymorphisme impliqué. Ainsi, dans le cas de polymorphisme de type microsatellite, on détectera avec avantage les produits de PCR selon leur taille en ayant recours aux techniques d'électrophorèse.

Lorsque le polymorphisme ne concerne que quelques nucléotides, voire un seul nucléotide, on aura plus spécialement recours, aux fins de différenciation des haplotypes de produits de PCR, aux techniques d'hybridation (analyse sur membrane à l'aide de sondes spécifiques des séquences d'haplotypes, SSOP ou Sequence Specific Oligonucleotide Probe), de migration différentielle des échantillons dénaturés (SSCP ou Single Strand Conformational Polymorphism), ou de séquençage. De manière générale, cette dernière technique est préférée compte tenu de la simplicité de sa réalisation.

L'invention fournit ainsi une technique simple et rapide d'établissement du profil génétique d'un grand nombre d'animaux à étudier, ce qui permet de déterminer les haplotypes et de sélectionner ceux d'intérêt en vue d'un élevage.

De plus, chaque type de gène pouvant être discriminé en utilisant des amorces présentant la spécificité requise et son appartenance à un système Rfp Y pouvant être établie, il est possible d'effectuer des études fondamentales plus complètes.

L'invention vise également un coffret ou trousse pour détecter le génotype du poulet ou autre oiseau d'élevage selon la méthode définie ci-dessus.

Ces coffrets ou trousses sont caractérisés en ce qu'ils comportent les réactifs nécessaires pour la réalisation d'au moins une PCR et du test de révélation.

En particulier, ils comportent les amorces pour la PCR, un témoin positif de la réaction, ainsi qu'une notice d'utilisation.

Les amorces se présentent sous forme lyophilisée ou en solution ou, selon le mode de détection, sur un support. Le support peut être, de manière classique, une plaque multipuits ou se présenter sous forme de puces à ADN.

L'invention vise en outre un système expérimental qui permet d'étudier la résistance au développement tumoral chez le poulet.

I1 s'agit de lignées d'animaux qui ont été triées génétiquement sur leurs caractéristiques du CMH. En fonction de ces caractéristiques, les lignées sont soit résistantes, soit sensibles vis-à-vis des tumeurs induites par des virus, comme le virus de la maladie de Marek. Cette sélection génétique, qui s'est dans un premier temps effectuée sur des critères sérologiques, a été ensuite poursuivie sur la base de l'étude du polymorphisme des gènes du CMH. Il s'agit d'un matériel génétique qui est parfaitement défini d'un point moléculaire, et constitue un outil précieux pour l'étude du polymorphisme des séquences de type microsatellite. Ce matériel, ainsi que le produit du croisement entre certaines des lignées entre elles, a été utilisé pour déterminer les séquences microsatellites du CMH qui sont polymorphes et pour évaluer si ce polymorphisme peut être corrélé avec les données de typage déjà disponibles pour ces lignées.

D'autres caractéristiques et avantages de l'invention sont exposés dans l'exemple qui suit.

### Exemple :

Etude d'haplotypes Rfp-Y du poulet à l'aide d'amorces microsatellitaires.
- amplification avec le Kit Expand™ High Fidelity PCR System
. Avec les amorces 17.5 R1/17.52
ADN génomique : 1 µg
Oligos prendre : 0,3 µM
dNTP : 8 µl
qsp H₂O 50 µl
On ajoute 50 µl de Mix 2 en mélangeant.
- Mix 2 :: 0,75 µl d'enzyme
10 µl TP10X avec MgCl₂
qsp H₂O 50 µl
Programme d'amplification :

| 30 Cycles | | | | |
|---|---|---|---|---|
| 94°C | 94°C | 65°C | 72°C | 4°C |
| 2' | 30" | 1' | 1' | **∞** |

Programme d'amplification :

| 30 Cycles | | | | |
|---|---|---|---|---|
| 94°C | 94°C | 60°C | 72°C | 4°C |
| 2' | 30' ' | 1' | 1' | ∞ |

- révélation par électrophorèse sur gel d'agarose ou par séquençage.

Le test a été appliqué à des haplotypes de poulet, sélectionnés sérologiquement pour le complexe Rfp-Y.

L'application pratique de cette méthode revient à soumettre les individus naturellement résistants au protocole décrit ci-dessus en prenant en compte les deux systèmes Rfp-Y et B du CMH et à ne sélectionner parmi des animaux à tester que ceux dont le profil correspond à celui des animaux résistants.

L'invention fournit ainsi les moyens de vérifier l'homogénéité des animaux et d'effectuer des sélections rigoureuses en prenant en compte chaque système du CMH, et dans ces systèmes les gènes recherchés.

## Revendications

1. Méthode de génotypage d'oiseaux d'élevage, permettant de contrôler les poulets résistants aux maladies viro-induites, **caractérisée en ce qu'**elle comprend
- l'amplification d'un échantillon d'acide nucléique provenant de l'animal à étudier à l'aide d'un ou de plusieurs couples d'amorces capables de s'hybrider spécifiquement avec l'acide nucléique d'une région polymorphe du système Rfp-Y du CMH desdits oiseaux, ces amorces étant élaborées à partir des molécules d'acides nucléiques du gène 17.5, et
- la détection des produits de PCR obtenus.

2. Méthode selon la revendication 1, **caractérisée en ce que** les amorces correspondent au couple :
17.52 : CAG GAT CTG CAC TGG CCA ATA
17.5, R1 : GAA TGG CGG TGC TTC CGT GCC TGG

3. Méthode selon la revendication 1 ou 2, **caractérisée en ce que** la détection des produits de PCR est effectuée par séquençage.

4. Coffret ou trousse pour le génotypage d'oiseaux d'élevages, **caractérisé en ce qu'**ils comportent les réactifs nécessaires pour la réalisation d'au moins une PCR et du test de révélation, selon la méthode de l'une quelconque des revendications 1 à 3.


### Annexe aux documents de la demande - listage des séquences déposé ultérieurement

SEQUENCE LISTING

<110> CENTRE NATIONAL DE LA RECHERCHE SCIENTIFIQUE (CNRS)
INSTITUT NATIONAL DE LA RECHERCHE AGRONOMIQUE (INRA)

<120> REACTIFS ET METHODES POUR LA DETECTION DE GENES LIES AU COMPLEXE MAJEUR
D'HISTOCOMPATIBILITE D'OISEAUX D'ELEVAGE, TELS QUE LE POULET

<130> CP/BB 59212-2062DIV

<140> EP 04 011 368.0
<141> 2004-05-13

<150> EP 98 955 723.6
<151> 1998-11-23

<150> PCT/FR97/14669
<151> 1998-11-23

<150> FR 97 14 669
<151> 1997-11-21

<160> 2

<170> PatentIn version 3.1

<210> 1
<211> 21
<212> DNA
<213> Artificial sequence

<220>
<223> Nucleotide primer
<400> 1
caggatctgc actggccaat a 21

<210>  2

<211>  24

<212>  DNA

<213>  Artificial Sequence

<220>

<223>  Nucleotide primer

<400>  2
gaatggcggt gcttccgtgc ctgg                                              24